# EUROPEAN PATENT SPECIFICATION

(11) **EP 3 111 429 B1**
(45) Date of publication and mention of the grant of the patent: **08.04.2020**
(21) Application number: 15755018.7
(22) Date of filing: 25.02.2015
(51) Int. Cl.: H04L 9/32, H04L 29/08, H04L 12/26, H04L 29/12, G01S 5/02, G01S 13/87, G06F 9/44, G08B 13/196, G08B 25/00, G08B 29/18, H04L 12/46, H04L 9/00, H04L 12/64, H04N 5/76, H04W 4/00, H04W 8/26, H04W 16/26, H04W 84/18, H04W 92/02, H04W 88/04

(54) **CORRELATION OF SENSORY INPUTS TO IDENTIFY UNAUTHORIZED PERSONS**
KORRELATION VON SENSORISCHEN EINGÄNGEN ZUR IDENTIFIZIERUNG VON UNBEFUGTEN PERSONEN
CORRÉLATION D'ENTRÉES SENSORIELLES POUR IDENTIFIER DES INDIVIDUS NON AUTORISÉS

(30) Priority: 28.02.2014 US 201461946054 P; 02.04.2014 US 201461973962 P; 20.08.2014 US 201414463765
(43) Date of publication of application: 04.01.2017
(73) Proprietor: Tyco Fire & Security GmbH, 8212 Neuhausen am Rheinfall (CH)
(72) Inventor: LOCKE, Robert B., Sonoma, California 95476 (US)
(74) Representative: Trinks, Ole
(86) International application number: PCT/US2015/017450
(87) International publication number: WO 2015/130744

(56) References cited:
- EP-A1- 2 037 426
- WO-A2-2007/002763
- WO-A2-2007/018523
- JP-A- 2005 292 942
- US-A- 4 581 634
- US-A1- 2007 094 716
- US-A1- 2007 252 001
- US-A1- 2008 083 018
- US-A1- 2014 253 706
- US-B2- 7 907 753

## Description

### CLAIM OF PRIORITY

This application claims priority under 35 U.S.C. §119(e) to provisional U.S. Patent Application 61/973,962, filed on April 2, 2014, entitled: "Wireless Sensor Network", and provisional U.S. Patent Application 61/946,054, filed on February 28, 2014, entitled: "Wireless Sensor Network", and utility U.S. Patent Application 14/463,765, filed on August 20, 2014, entitled: "Correlation of Sensory Inputs to Identify Unauthorized Persons".

### Background

This description relates to operation of sensor networks such as those used for security, intrusion and alarm systems installed on commercial or residential premises.

It is common for businesses and homeowners to have a security system for detecting alarm conditions at their premises and signaling the conditions to a monitoring station or to authorized users of the security system. Security systems often include an intrusion detection panel that is electrically or wirelessly connected to a variety of sensors. Those sensors types typically include motion detectors, cameras, and proximity sensors (used to determine whether a door or window has been opened). Typically, such systems receive a very simple signal (electrically open or closed) from one or more of these sensors to indicate that a particular condition being monitored has changed or become unsecure.

Typical intrusion systems can be set up to monitor entry doors in a building. When the door is secured, the proximity sensor senses a magnetic contact and produces an electrically closed circuit. When the door is opened, the proximity sensor opens the circuit, and sends a signal to the panel indicating that an alarm condition has occurred (e.g., an opened entry door). Government entities, companies, academic institutions, etc. issue credentials to employees, contractors, students, etc. to control access to buildings and facilities, indoors and outdoors. Individuals who bypass security systems to gain access, either intentionally or unintentionally, are difficult to identify and locate.

JP 2005292942A discloses a security system in which a security controller counts monitored objects in a security zone according to image data picked up by imaging means. A terminal communicates with portable terminals of the monitored objects to count the number of portable terminals. The number of portable terminals is compared with the number of monitored objects in order to determine if there is an intruder present.

EP 2037426 A1 discloses a security system in which a mobile object included in an image is detected by way of a camera. An ID number is associated with the mobile object. Permitted activities and prohibited activities are defined according to authorization corresponding to the authentication information. An alarm is generated if the mobile object performs an activity which is considered to be prohibited.

WO 2007/002763 A2 discloses a video surveillance system which extracts video primitives and extracts event occurrences from the video primitives using event discriminators. The system may determine whether an authorized person is located in a room by comparing the number of people against a number of card swipes used to enter the room.

WO 2007/018523 discloses outputs of monocular and stereo video trackers which are fused with RFID and localization systems and biometric identification systems. The system may be used to continuously track multiple humans with their identities in a larger area.

US 2008/0083018 A1 discloses a system for integrating security and access for facilities and information systems including a computer server, information systems, and facility protection systems. The system may store information defining users and their access privileges to information services and to areas of facilities controlled by access control system systems. The facility may automatically respond to security risks.

### SUMMARY

Prior solutions regarding credentials have focused on technologies such as video surveillance to address access problems. Once a person has gained access, however, it is difficult to impossible to distinguish between those with valid credentials and those without valid credentials.

The invention is defined by the system of independent claim 1. Dependent claims define preferred embodiments.

According to an aspect of a system for physical intrusion detection/alarm monitoring includes one or more computing devices, including processor devices and memory in communication with the processor devices. The computing device are configured to: receive, from reader devices, sensory inputs from credentials or badges, with the reader being within a monitored premises; receive video information from cameras and other image capture devices disposed throughout the premises; continually correlate the received sensory inputs from the credentials or badges with the received video by determining, from the received sensory inputs, the number of unique credentials or badges within one or more areas of the monitored premises and determining, from the received video, the number of individuals within the one or more areas of the monitored premises; and continually track the credentials or badges such that, when one or more of the tracked credentials or badges and a corresponding number of individuals departs the one or more areas, the system is configured to stop tracking those one or more credentials or badges, while continually tracking any remaining badges or credentials and a non-corresponding number of individuals, in order to isolate a possible non-credentialed individual appearing in the received video.

One or more of the following are some of the embodiments within the scope of this aspect. system is further configured to apply one or more algorithms to detect the presence of a possible non-credentialed individual and track at least the non-credentialed individual and produce an alert to send to a control center to alert authorities to the location of the non-credentialed individual. The system is further configured to apply video recognition to identify the number of people in a certain area and correlate that data with data from one or more remote badge readers to identify the appropriately number of badged individuals in a group of individuals within a monitored area. The system is further configured to determine a mismatch between the number of individuals in the area and a number of read badges or credentials. The system is further configured to continually track all individuals and their movements throughout the premises, correlate those movements with different readings of valid credentials or badges to isolate one or more non-credentialed individuals. The one or more computing devices include an application layer that executes routines to provide node functions, with the application layer operating under an operating system allows for loading and execution of individual node functions after an initial booting of the device, but without requiring a rebooting of the device to execute routines that are dynamically changed after the initial booting of the device. At least some of the nodes are cameras and others are credential readers. Certain of the nodes are configured to apply video recognition to frames of captured video to recognize features that correspond to individuals appearing in the captured frames and determine a number of people within the image. In one or more of the certain nodes, the one or more of the certain nodes are configured to change the video recognition algorithm that is applied to find features that correspond to a number of individuals. The system is further configured to correlate paths taken by different individuals with different readings of valid credentials or badges from the same or different sets of cameras/readers.

One or more aspects may provide one or more of the following advantages.

By correlating sensory input from credentials or badges that use technologies such as RFID, Bluetooth low energy (BLE), MAC addresses from cell phones, NFC, etc. with video; individuals without valid credentials can be identified and tracked. Video recognition is used to identify the number of people in a certain area. A remote reading badge is used to identify the appropriately badged employees by tracking the personnel movement and correlating that with the movement of the valid ID. Once the non-badged individual is segregated from the valid personnel, biometrics such as face, iris or just video recognition are used to track the individual(s) and allow authorities to intervene.

The details of one or more embodiments of the invention are set forth in the accompanying drawings and the description below. Other features, objects, and advantages of the invention is apparent from the description and drawings, and from the claims.

### DESCRIPTION OF DRAWINGS

FIG. 1 is a schematic diagram of an exemplary networked security system.
FIG. 2 is a flow chart of a correlation algorithm.
FIG. 3 is flow chart of an example tracking process.
FIG. 4 is a block diagram of components of an example networked security system.

### DETAILED DESCRIPTION

Described herein are examples of network features that may be used in various contexts including, but not limited to, security/intrusion and alarm systems. Example security systems may include an intrusion detection panel that is electrically or wirelessly connected to a variety of sensors. Those sensors types may include motion detectors, cameras, and proximity sensors (used, e.g., to determine whether a door or window has been opened). Typically, such systems receive a relatively simple signal (electrically open or closed) from one or more of these sensors to indicate that a particular condition being monitored has changed or become unsecure.

For example, typical intrusion systems can be set-up to monitor entry doors in a building. When a door is secured, a proximity sensor senses a magnetic contact and produces an electrically closed circuit. When the door is opened, the proximity sensor opens the circuit, and sends a signal to the panel indicating that an alarm condition has occurred (e.g., an opened entry door).

Data collection systems are becoming more common in some applications, such as home safety monitoring. Data collection systems employ wireless sensor networks and wireless devices, and may include remote server-based monitoring and report generation. As described in more detail below, wireless sensor networks generally use a combination of wired and wireless links between computing devices, with wireless links usually used for the lowest level connections (e.g., end-node device to hub/gateway). In an example network, the edge (wirelessly-connected) tier of the network is comprised of resource-constrained devices with specific functions. These devices may have a small-to-moderate amount of processing power and memory, and may be battery powered, thus requiring that they conserve energy by spending much of their time in sleep mode. A typical model is one where the edge devices generally form a single wireless network in which each end-node communicates directly with its parent node in a hub-and-spoke-style architecture. The parent node may be, e.g., an access point on a gateway or a sub-coordinator which is, in turn, connected to the access point or another sub-coordinator.

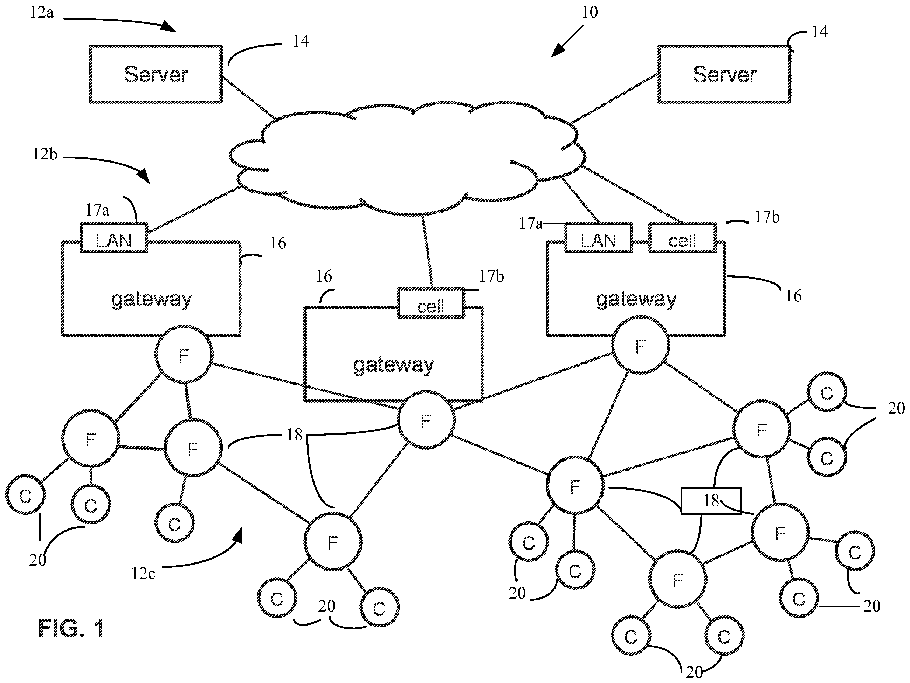

Referring now to FIG. 1, an exemplary (global) distributed network 10 topology for a Wireless Sensor Network (WSN) is shown. In FIG. 1 the distributed network 10 is logically divided into a set of tiers or hierarchical levels 12a-12c.

In an upper tier or hierarchical level 12a of the network are disposed servers and/or virtual servers 14 running a "cloud computing" paradigm that are networked together using well-established networking technology such as Internet protocols or which can be private networks that use none or part of the Internet. Applications that run on those servers 14 communicate using various protocols such as for Web Internet networks XML/SOAP, RESTful web service, and other application layer technologies such as HTTP and ATOM. The distributed network 10 has direct links between devices (nodes) as shown and discussed below.

The distributed network 10 includes a second logically divided tier or hierarchical level 12b, referred to here as a middle tier that involves gateways 16 located at central, convenient places inside individual buildings and structures. These gateways 16 communicate with servers 14 in the upper tier whether the servers are stand-alone dedicated servers and/or cloud based servers running cloud applications using web programming techniques. The middle tier gateways 16 are also shown with both local area network 17a (e.g., Ethernet or 802.11) and cellular network interfaces 17b.

The distributed network topology also includes a lower tier (edge layer) 12c set of devices that involve fully-functional sensor nodes 18 (e.g., sensor nodes that include wireless devices, e.g., transceivers or at least transmitters, which in FIG. 1 are marked in with an "F") as well as constrained wireless sensor nodes or sensor end-nodes 20 (marked in the FIG. 1 with "C"). In some embodiments wired sensors (not shown) can be included in aspects of the distributed network 10.

Constrained computing devices 20 as used herein are devices with substantially less persistent and volatile memory other computing devices, sensors in a detection system. Currently examples of constrained devices would be those with less than about a megabyte of flash/persistent memory, and less than 10-20 kbytes of RAM/volatile memory). These constrained devices 20 are configured in this manner; generally due to cost/physical configuration considerations.

In a typical network, the edge (wirelessly-connected) tier of the network is comprised of highly resource-constrained devices with specific functions. These devices have a small-to-moderate amount of processing power and memory, and often are battery powered, thus requiring that they conserve energy by spending much of their time in sleep mode. A typical model is one where the edge devices generally form a single wireless network in which each end-node communicates directly with its parent node in a hub-and-spoke-style architecture. The parent node may be, e.g., an access point on a gateway or a sub-coordinator which is, in turn, connected to the access point or another sub-coordinator.

Each gateway is equipped with an access point (fully functional node or "F" node) that is physically attached to that access point and that provides a wireless connection point to other nodes in the wireless network. The links (illustrated by lines not numbered) shown in FIG. 1 represent direct (single-hop network layer) connections between devices. A formal networking layer (that functions in each of the three tiers shown in FIG. 1) uses a series of these direct links together with routing devices to send messages (fragmented or non-fragmented) from one device to another over the network.

The WSN 10 implements a state machine approach to an application layer that runs on the lower tier devices 18 and 20. Discussed below is an example of a particular implementation of such an approach. States in the state machine are comprised of sets of functions that execute in coordination, and these functions can be individually deleted or substituted or added to in order to alter the states in the state machine of a particular lower tier device.

The WSN state function based application layer uses an edge device operating system (not shown, but such as disclosed in the above mentioned provisional application) that allows for loading and execution of individual functions (after the booting of the device) without rebooting the device (so-called "dynamic programming"). In other implementations, edge devices could use other operating systems provided such systems allow for loading and execution of individual functions (after the booting of the device) preferable without rebooting of the edge devices.

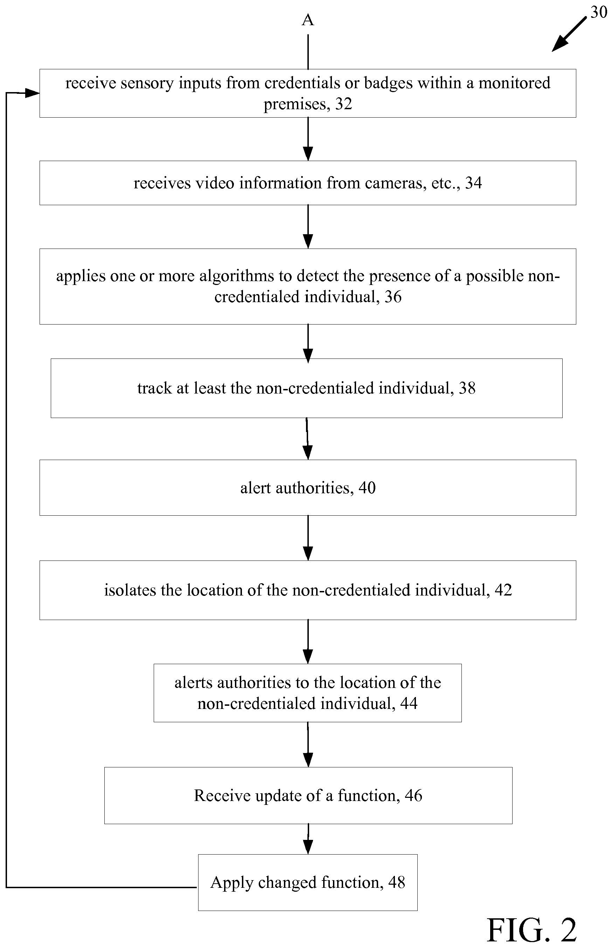

Referring now to FIG. 2 a process 30 that executes on one or more computers disposed within the distributed network 10 is shown. In alternative arrangements, the process 30 can be used in any other arrangement besides the distributed network 10 described above provided that video data and sensed credential data are supplied to the computer(s). The process 30 correlates sensory input from credentials or badges that use technologies such as RFID, Bluetooth low energy (BLE), MAC addresses from cell phones, NFC, etc. with captured video.

The process 30 executes on the one or more computers, and receives 32 sensory inputs from credentials or badges within a monitored premises. Sensors such as some of the nodes in FIG. 1 sense the presence of a credential tag typically carried by an individual passing through a range of the sensor. These sensors receive signals from badge/tag devices that incorporate circuitry operative using radio frequency identification (RFID), Bluetooth® low energy peer to peer devices, MAC addresses from cell phones, and near field communication (NFC) devices operative using a set of standards for smartphones and similar devices to establish radio communication with each other by touching them together or bringing them into proximity, etc. that are dispersed throughout a monitored premises. The process 30 also receives 34 video information from cameras and other image capture devices that are disposed throughout the premises. The process 30 continually applies one or more algorithms to detect the presence of a possible non-credentialed individual to continually correlate the received sensory inputs from these credentials or badges, etc. with the received video.

The process 30 seeks to track individuals, especially individuals without valid credentials or badges for a particular monitored area. The process 30 applies one or more algorithms that detect the presence of a possible non-credentialed individual, tracks 38 at least the non-credentialed individual, alerts 40 authorities of the presence of a non-credentialed individual within the premises, and continually processing of inputs to isolate tracking of the non-credentialed individual to a particular, e.g., pinpoint location and alert 42 authorities to the isolated location of the non-credentialed individual.

During processing in FIG. 2, various ones of the nodes in FIG. 1 can be brought to bear on tracking of the non-credentialed individual or generally tracking of credentialed individuals. Thus, the servers of FIG. 1 or other systems can generate updates to functions (not shown) that are performed at the lower tiers, these nodes can receive 46 these new or updated functions and apply 48 the changed function to processing of FIG. 2.

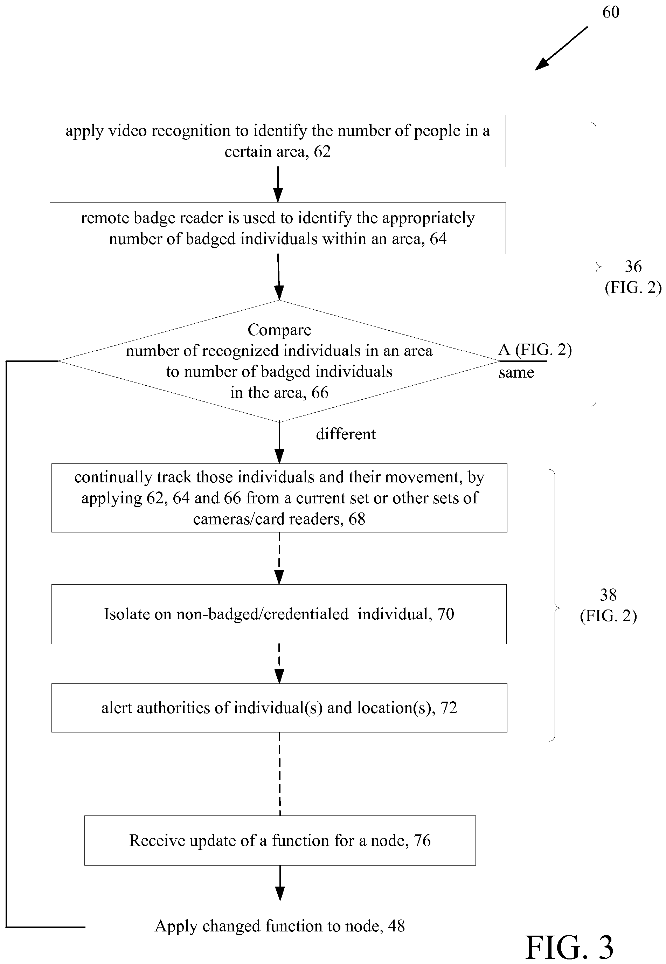

Referring now to FIG. 3 an example of algorithmic processing 60 for the tracking process 30 of FIG. 3, is shown. Cameras dispersed without the premises being monitored. Generally, the processing 36 of FIG. 2 is shown with details. One camera in an area captures video and applies 62 video recognition to frames of captured video. The video recognition is used to recognize features that correspond to, e.g., individuals appearing in the captured frames. The video recognition determines a number of people with the image, where the image is correlated to the area captured by the camera. At this junction the video recognition that is applied can be rather coarse to conserve processing or allow such processing to be performed at nodes within the lower tier, as it merely needs to find features that correspond to a number of individuals.

A remote badge reader that can be one of the nodes in the network 10 has a range that coincides, or overlaps or otherwise can be correlated to the area that was captured in the video frames. This remote badge reader and camera can be considered as a current set.

The computer(s) receives 64 badge/tag data from those individuals that are within the range of operation of the badge reader. The remote badge reader and/or computer(s) determines 66 the number of badged individuals that pass through the region within the range of operation of the remote badge reader. If the area captured by the remote badge reader coincides with the area captured by the camera, this data can be processed, otherwise they may be some correlation (not shown) need to correlate the area captured in the video frames with the area within the range of the card reader.

The computer compares the number of recognized individuals in frames to the number of received badges from the reader. If the process 60 determines that there is a mismatch between the number of individuals in the area and a number of badges (or credentials), the process 60 continues (generally, the processing 38 of FIG. 2) to track 68 all of those individuals and their movements throughout the premises by continually applying 62 video recognition to frames of captured video, receive 64 badge/tag and determining 66 the number of badged individuals that pass through the region within the range of badge readers with respect to the number of recognized individuals, using either the current set of cameras/readers or different sets of cameras/readers, as needed. At this juncture, the process could send requests for updated algorithms, as is discussed below for both the overall process (as in FIG. 2) or to update nodes for different node-level processing and sensing (FIG. 3).

The process 60 correlates the paths taken by different individuals with different readings of valid credentials or badges from the same or different sets of cameras/readers.

For example, at this junction more sophisticated recognition algorithms, e.g., facial recognition, etc. can be used. In addition, processing algorithms can be sent to other nodes in the network to train process on the tracked individuals where the nodes that are send these algorithms are selected based on an estimation or prediction of direction/paths of travel through the premises.

At some point as individuals come and go, a non-badged/credentialed individual can be isolated individually or to a small group, and then the process will focus tracking on that individual. At any point in processing where there is a discrepancy an alarm can be raised. As the process detects the presence of new individuals and detects the departure of previously tracked individuals from the group of individuals, the process is still continually tracking the one or more individuals without valid credentials. Newly added individuals can be recognized in the video captured, especially if more intensive algorithms are used, and departing individuals can be noted by a valid reading of their tags/credentials. If an individual departs without a valid tag read, when is should have been read, that person is most likely the non-credentialed individual.

As previously mentioned, using the network 10 of FIG. 1, it is possible during processing that various ones of the nodes in FIG. 1 are brought to bear on tracking of FIG. 2. Thus, the servers of FIG. 1 or other systems can generate updates to functions (not shown) that are performed at the lower tiers e.g., such nodes. These nodes at the lower tier receive 46 these new or updated functions and apply 48 the changed function to the processing performed by the nodes within processing of FIG. 2.

Examples of updated processing include sending more sophisticated recognition algorithms to video cameras or nodes that process the video information. Other examples are that certain ones of the nodes in FIG. 1 can be IP address reading sensors that are brought to bear on tracking of the non-credentialed individual or generally tracking of credentialed individuals.

Thus, the servers of FIG. 1 or other systems can generate updates to functions (not shown) that are performed at the lower tiers, these nodes can receive 46 these new or updated functions and apply 48 the changed function to processing of FIG. 2. As previously tracked individuals the depart, card readers can determine/sense credentials and the process can determine if they were validly credentialed and if so, terminate tracking on those departed individuals. On the other hand as new tracked individuals join, card readers can determine/sense credentials and the process can determine if they are validly credentialed and if so, terminate tracking on those new individuals. At some point the non-credentialed individual can be isolated to one or a few individuals, and his/their location(s) identified. The process produces and sends a message that notifies authorities to physically intervene with the message including available location information.

The nodes may be implemented using any appropriate type of computing device, such as a mainframe work station, a personal computer, a server, a portable computing device, or any other type of intelligent device capable of executing instructions, connecting to a network, and forwarding data packets through the network. The nodes can execute any appropriate computer programs to generate, receive, and transmit data packets for use on the network.

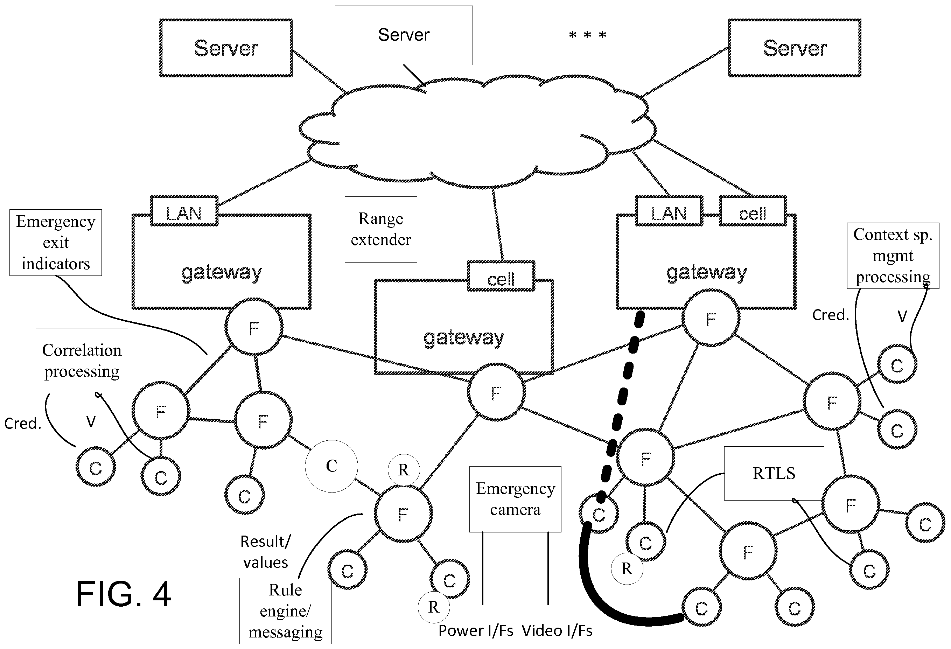

FIG. 4 shows an example of a security system having features of the WSN described with respect to FIGS. 1 to 3 and having the various functionalities described herein. As shown in FIG. 4, correlation processing receives inputs from certain constrained nodes (although these can also be fully functional nodes). These inputs may include credential information and video information, and the correlation processing may produce correlated results that are sent over the network. Context management processing receives inputs from certain constrained nodes (although these can also be fully functional nodes) e.g., credential information and video and grouping information, and performs context processing with results sent over the network. The network supports operation of emergency exit indicators; emergency cameras as well as distributed rule processing and rule engine/messaging processing. Range extenders are used with e.g., gateways, and a real time location system receives inputs from various sensors (e.g., constrained type) as shown. Servers interface to the WSN via a cloud computing configuration and parts of some networks can be run as sub-nets.

The sensors provide in addition to an indication that something is detected in an area within the range of the sensors, detailed additional information that can be used to evaluate what that indication may be without the intrusion detection panel being required to perform extensive analysis of inputs to the particular sensor.

For example, a motion detector could be configured to analyze the heat signature of a warm body moving in a room to determine if the body is that of a human or a pet. Results of that analysis would be a message or data that conveys information about the body detected. Various sensors thus are used to sense sound, motion, vibration, pressure, heat, images, and so forth, in an appropriate combination to detect a true or verified alarm condition at the intrusion detection panel.

Recognition software can be used to discriminate between objects that are a human and objects that are an animal; further facial recognition software can be built into video cameras and used to verify that the perimeter intrusion was the result of a recognized, authorized individual. Such video cameras would comprise a processor and memory and the recognition software to process inputs (captured images) by the camera and produce the metadata to convey information regarding recognition or lack of recognition of an individual captured by the video camera. The processing could also alternatively or in addition include information regarding characteristic of the individual in the area captured/monitored by the video camera. Thus, depending on the circumstances, the information would be either metadata received from enhanced motion detectors and video cameras that performed enhanced analysis on inputs to the sensor that gives characteristics of the perimeter intrusion or a metadata resulting from very complex processing that seeks to establish recognition of the object.

Sensor devices can integrate multiple sensors to generate more complex outputs so that the intrusion detection panel can utilize its processing capabilities to execute algorithms that analyze the environment by building virtual images or signatures of the environment to make an intelligent decision about the validity of a breach.

Memory stores program instructions and data used by the processor of the intrusion detection panel. The memory may be a suitable combination of random access memory and read-only memory, and may host suitable program instructions (e.g. firmware or operating software), and configuration and operating data and may be organized as a file system or otherwise. The stored program instruction may include one or more authentication processes for authenticating one or more users. The program instructions stored in the memory of the panel may further store software components allowing network communications and establishment of connections to the data network. The software components may, for example, include an internet protocol (IP) stack, as well as driver components for the various interfaces, including the interfaces and the keypad . Other software components suitable for establishing a connection and communicating across network will be apparent to those of ordinary skill.

Program instructions stored in the memory, along with configuration data may control overall operation of the panel.

The monitoring server includes one or more processing devices (e.g., microprocessors), a network interface and a memory (all not illustrated). The monitoring server may physically take the form of a rack mounted card and may be in communication with one or more operator terminals (not shown). An example monitoring server is a SURGARD™ SG-System III Virtual, or similar system.

The processor of each monitoring server acts as a controller for each monitoring server , and is in communication with, and controls overall operation, of each server. The processor may include, or be in communication with, the memory that stores processor executable instructions controlling the overall operation of the monitoring server. Suitable software enable each monitoring server to receive alarms and cause appropriate actions to occur. Software may include a suitable Internet protocol (IP) stack and applications/clients.

Each monitoring server of the central monitoring station may be associated with an IP address and port(s) by which it communicates with the control panels and/or the user devices to handle alarm events, etc. The monitoring server address may be static, and thus always identify a particular one of monitoring server to the intrusion detection panels. Alternatively, dynamic addresses could be used, and associated with static domain names, resolved through a domain name service.

The network interface card interfaces with the network to receive incoming signals, and may for example take the form of an Ethernet network interface card (NIC). The servers may be computers, thin-clients, or the like, to which received data representative of an alarm event is passed for handling by human operators. The monitoring station may further include, or have access to, a subscriber database that includes a database under control of a database engine. The database may contain entries corresponding to the various subscriber devices/processes to panels like the panel that are serviced by the monitoring station.

All or part of the processes described herein and their various modifications (hereinafter referred to as "the processes") can be implemented, at least in part, via a computer program product, i.e., a computer program tangibly embodied in one or more tangible, physical hardware storage devices that are computer and/or machine-readable storage devices for execution by, or to control the operation of, data processing apparatus, e.g., a programmable processor, a computer, or multiple computers. A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a network.

Actions associated with implementing the processes can be performed by one or more programmable processors executing one or more computer programs to perform the functions of the calibration process. All or part of the processes can be implemented as, special purpose logic circuitry, e.g., an FPGA (field programmable gate array) and/or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only storage area or a random access storage area or both. Elements of a computer (including a server) include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, e.g., magnetic, magneto-optical disks, or optical disks.

Tangible, physical hardware storage devices that are suitable for embodying computer program instructions and data include all forms of non-volatile storage, including by way of example, semiconductor storage area devices, e.g., EPROM, EEPROM, and flash storage area devices; magnetic disks, e.g., internal hard disks or removable disks; magneto-optical disks; and CD-ROM and DVD-ROM disks and volatile computer memory, e.g., RAM such as static and dynamic RAM, as well as erasable memory, e.g., flash memory.

In addition, the logic flows depicted in the figures do not require the particular order shown, or sequential order, to achieve desirable results. In addition, other actions may be provided, or actions may be eliminated, from the described flows, and other components may be added to, or removed from, the described systems. Likewise, actions depicted in the figures may be performed by different entities or consolidated.

Elements of different embodiments described herein may be combined to form other embodiments not specifically set forth above. Elements may be left out of the processes, computer programs, Web pages, etc. described herein without adversely affecting their operation. Furthermore, various separate elements may be combined into one or more individual elements to perform the functions described herein.

## Claims

1. A system (10) for physical intrusion detection/alarm monitoring comprises: one or more computing devices, comprising processor devices and memory in communication with the processor devices, configured to:
- receive (32), from remote badge reader devices, sensory inputs from credentials or badges, with the reader devices being within a monitored premises;
- receive (34) video information from cameras and other image capture devices disposed throughout the premises;
- continually correlate (36) the received sensory inputs from the credentials or badges with the received video by determining (66), from the received sensory inputs, the number of credentials or badges within one or more areas of the monitored premises and determining (66), from the received video, the number of individuals within the one or more areas of the monitored premises;
- apply one or more recognition algorithms to detect the presence of a number of individuals in the received video;
- isolate (70) a possible non credentialed individual appearing in the received video by:
-. applying (62) video recognition to identify the number of people in a certain area and correlate that data with data from one or more of the remote badge readers to identify the appropriately number of badged individuals in a group of individuals within a monitored area, and by
-. continually tracking (38) the credentials or badges such that, when one or more of the tracked credentials or badges and a corresponding number of individuals departs the one or more areas, the system is configured to stop tracking those one or more credentials or badges, while continually tracking any remaining badges or credentials and a non-corresponding number of individuals; and
- produce an alert (44) to send to a control center to alert authorities to the location of an isolated non credentialed individual.

2. The system (10) of claim 1 further configured to:
determine a mismatch between the number of individuals in the area and a number of read badges or credentials.

3. The system (10)of claim 2 further configured to:
continually track (68) all individuals and their movements throughout the premises;
correlate those movements with different readings of valid credentials or badges to isolate one or more non-credentialed individuals.

4. The system (10) of claim 1 wherein the one or more computing devices include an application layer that executes routines to provide node functions, with the application layer operating under an operating system allows for loading and execution of individual node functions after an initial booting of the device, but without requiring a rebooting of the device to execute routines that are dynamically changed after the initial booting of the device.

5. The system (10) of claim 1 wherein at least some of the nodes are cameras and others are credential readers.

6. The system (10) of claim 1 wherein certain of the nodes are configured to: apply video recognition to frames of captured video to recognize features that correspond to individuals appearing in the captured frames; and determine a number of people within the image.

7. The system (10) of claim 6 wherein in one or more of the certain nodes, the one or more of the certain nodes are configured to:
change the video recognition algorithm that is applied to find features that correspond to a number of individuals.

8. The system (10) of claim 1 configured to:
correlate paths taken by different individuals with different readings of valid credentials or badges from the same or different sets of cameras/readers.

## Patentansprüche

1. System (10) zur physischen Intrusionsdetektion/Alarmüberwachung, das Folgendes umfasst: ein oder mehrere Computervorrichtungen, umfassend Prozessorvorrichtungen und Speicher, der in Kommunikation mit den Prozessorvorrichtungen steht, ausgelegt für:
- Empfangen (32), von entfernten Ausweislesevorrichtungen, von sensorischen Eingaben von Berechtigungsnachweisen oder Ausweisen, wobei sich die Lesevorrichtungen in überwachten Räumlichkeiten befinden;
- Empfangen (34) von Videoinformationen von Kameras und anderen Bilderfassungsvorrichtungen, die in den gesamten Räumlichkeiten angeordnet sind;
- kontinuierliches Korrelieren (36) der empfangenen sensorischen Eingaben von den Berechtigungsnachweisen oder Ausweisen mit den empfangenen Videoinformationen durch Bestimmen(66), anhand der empfangenen sensorischen Eingaben, der Anzahl von Berechtigungsnachweisen oder Ausweisen in einem oder mehreren Bereichen der überwachten Räumlichkeiten und Bestimmen (66), anhand der empfangenen Videoinformationen, der Anzahl von Einzelpersonen in den ein oder mehreren Bereichen der überwachten Räumlichkeiten;
- Anwenden von einem oder mehreren Erkennungsalgorithmen, um die Anwesenheit einer Anzahl von Einzelpersonen in den empfangenen Videoinformationen zu detektieren;
- Isolieren (70) einer möglichen Einzelperson ohne Berechtigungsnachweis, die in den empfangenen Videoinformationen erscheint, durch:
- Anwenden (62) einer Videoerkennung, um die Anzahl von Personen in einem bestimmten Bereich zu identifizieren und diese Daten mit Daten von einem oder mehreren der entfernten Ausweisleser zu korrelieren, um die entsprechende Anzahl von ausgewiesenen Einzelpersonen in einer Gruppe von Einzelpersonen in einem überwachten Bereich zu identifizieren, und durch
- kontinuierliches Verfolgen (38) der Berechtigungsnachweise oder Ausweise, sodass, wenn einer oder mehrere der verfolgten Berechtigungsnachweise oder Ausweise und eine entsprechende Anzahl von Einzelpersonen den einen oder die mehreren Bereiche verlässt, das System dafür ausgelegt ist, die Verfolgung dieser ein oder mehreren Berechtigungsnachweise oder Ausweise zu beenden, während alle verbleibenden Ausweise oder Berechtigungsnachweise und eine nicht-entsprechende Anzahl von Einzelpersonen weiterhin kontinuierlich verfolgt werden; und
- Generieren eines Alarms (44), der an eine Steuerungszentrale gesendet werden soll, um zuständige Stellen auf den Standort einer isolierten Einzelperson ohne Berechtigungsnachweis aufmerksam zu machen.

2. System (10) nach Anspruch 1, welches ferner ausgelegt ist für:
Bestimmen einer Nichtübereinstimmung der Anzahl von Einzelpersonen in dem Bereich und einer Anzahl von gelesenen Ausweisen oder Berechtigungsnachweisen.

3. System (10) nach Anspruch 2, welches ferner ausgelegt ist für:
kontinuierliches Verfolgen (68) von allen Einzelpersonen und deren Bewegungen in den gesamten Räumlichkeiten;
Korrelieren dieser Bewegungen mit unterschiedlichen Ablesewerten von gültigen Berechtigungsnachweisen oder Ausweisen, um eine oder mehrere Einzelpersonen ohne Berechtigungsnachweis zu isolieren.

4. System (10) nach Anspruch 1, wobei die ein oder mehreren Computervorrichtungen eine Anwendungsebene aufweisen, die Routinen ausführt, um Knotenfunktionen bereitzustellen, wobei die Anwendungsebene, die unter einem Betriebssystem betrieben wird, das Laden und die Ausführung einzelner Knotenfunktionen nach einem ersten Hochfahren der Vorrichtung ermöglicht, jedoch ohne dass ein erneutes Hochfahren der Vorrichtung erforderlich ist, um Routinen auszuführen, die nach dem ersten Hochfahren der Vorrichtung dynamisch geändert werden.

5. System (10) nach Anspruch 1, wobei wenigstens einige der Knoten Kameras sind und andere Berechtigungsnachweisleser sind.

6. System (10) nach Anspruch 1, wobei bestimmte Knoten ausgelegt sind für: Anwenden einer Videoerkennung auf Rahmen von erfassten Videoinformationen, um Merkmale zu erkennen, die Einzelpersonen entsprechen, welche in den erfassten Rahmen erscheinen; und Bestimmen einer Anzahl von Personen in dem Bild.

7. System (10) nach Anspruch 1, wobei in einem oder mehreren der bestimmten Knoten der eine oder die mehreren bestimmten Knoten ausgelegt sind für:
Ändern des Videoerkennungsalgorithmus, der angewendet wird, um Merkmale zu finden, die einer Anzahl von Einzelpersonen entsprechen.

8. System (10) nach Anspruch 1, welches ausgelegt ist für:
Korrelieren von Wegen, die von unterschiedlichen Einzelpersonen mit unterschiedlichen Ablesewerten gültiger Berechtigungsnachweise oder Ausweise von denselben oder unterschiedlichen Sätzen von Kameras/Lesern genommen werden.

## Revendications

1. Système (10) de détection d'intrusion physique/ de surveillance d'alarme, comprenant : un ou plusieurs dispositifs informatiques, comprenant des dispositifs de processeur et une mémoire en communication avec les dispositifs de processeur, configurés pour :
- recevoir (32), depuis des dispositifs de lecture de badge à distance, des entrées sensorielles provenant de pièces d'identité ou de badges, les dispositifs de lecture se trouvant à l'intérieur de locaux surveillés ;
- recevoir (34) des informations vidéo provenant de caméras et d'autres dispositifs de capture d'image disposés partout dans les locaux ;
- corréler en continu (36) les entrées sensorielles reçues provenant des pièces d'identité ou des badges avec la vidéo reçue en déterminant (66), à partir des entrées sensorielles reçues, le nombre de pièces d'identité ou de badges à l'intérieur d'une ou de plusieurs zones des locaux surveillés et déterminer (66), à partir de la vidéo reçue, le nombre d'individus à l'intérieur des une ou plusieurs zones des locaux surveillés ;
- appliquer un ou plusieurs algorithmes de reconnaissance pour détecter la présence d'un nombre d'individus dans la vidéo reçue ;
- isoler (70) un individu sans pièce d'identité possible apparaissant dans la vidéo reçue par :
- l'application (62) d'une reconnaissance vidéo pour identifier le nombre de personnes dans une certaine zone et corréler ces données avec des données provenant d'un ou de plusieurs des lecteurs de badge à distance pour identifier le nombre approprié d'individus portant un badge dans un groupe d'individus à l'intérieur d'une zone surveillée, et par
- le suivi en continu (38) des pièces d'identité ou des badges de telle sorte que quand un(e) ou plusieurs des pièces d'identité ou des badges suivis et un nombre correspondants d'individus quittent les une ou plusieurs zones, le système est configuré pour arrêter le suivi de ces un(e) ou plusieurs pièces d'identité ou badges, tout en suivant en continu d'éventuels badges ou pièces d'identité restants et un nombre non correspondant d'individus ; et
- produire une alerte (44) à envoyer à un centre de commande pour alerter les autorités de l'emplacement d'un individu isolé sans pièce d'identité.

2. Système (10) selon la revendication 1, en outre configuré pour :
déterminer une non-concordance entre le nombre d'individus dans la zone et un nombre de badges ou de pièces d'identité lus.

3. Système (10) selon la revendication 2, en outre configuré pour :
suivre en continu (68) tous les individus et leurs mouvement partout dans les locaux ;
corréler ces mouvements avec différents relevés de pièces d'identité ou de badges valables pour isoler un ou plusieurs individus sans pièce d'identité.

4. Système (10) selon la revendication 1, dans lequel les un ou plusieurs dispositifs informatiques comprennent une couche d'application qui exécute des routines pour fournir des fonctions de nœud, la couche d'application fonctionnant sous un système d'exploitation permettant de charger et d'exécuter des fonctions de nœud individuelles après un démarrage initial du dispositif mais sans nécessiter un redémarrage du dispositif pour exécuter des routines qui sont modifiées de manière dynamique après le démarrage initial du dispositif.

5. Système (10) selon la revendication 1, dans lequel au moins certains des nœuds sont des caméras et d'autres sont des lecteurs de pièces d'identité.

6. Système (10) selon la revendication 1, dans lequel certains des nœuds sont configurés pour : appliquer une reconnaissance vidéo à des trames de vidéo capturée pour reconnaître des particularités qui correspondent à des individus apparaissant dans les trames capturées ; et déterminer un nombre de personnes à l'intérieur de l'image.

7. Système (10) selon la revendication 6, dans lequel, dans un ou plusieurs des certains nœuds, les un ou plusieurs des certains nœuds sont configurés pour : changer l'algorithme de reconnaissance vidéo qui est appliqué pour trouver des particularités qui correspondent à un nombre d'individus.

8. Système (10) selon la revendication 1, configuré pour : corréler des chemins empruntés par différents individus avec différents relevés de pièces d'identité ou de badges valables provenant d'ensembles de caméras/lecteurs semblables ou différents.
